# EUROPEAN PATENT SPECIFICATION

(11) **EP 2 285 388 B1**
(45) Date of publication and mention of the grant of the patent: **26.03.2014**
(21) Application number: 09726179.6
(22) Date of filing: 26.03.2009
(51) Int. Cl.: A61K 31/7048, A61P 3/10

(54) **USE OF OLEUROPEIN AND DERIVATIVES IN THE TREATMENT OF TYPE 2 DIABETES MELLITUS AND PATHOLOGIES ASSOCIATED WITH PROTEIN AGGREGATION PHENOMENA**
VERWENDUNG VON OLEUROPEIN UND DERIVATEN BEI DER BEHANDLUNG VON TYP 2 DIABETES MELLITUS UND PATHOLOGIEN IM ZUSAMMENHANG MIT PROTEINAGGREGATIONSPHÄNOMENEN
UTILISATION D OLEUROPÉINE ET DE DÉRIVÉS DE CELLE-CI DANS LE TRAITEMENT DU DIABÈTE SUCRÉ DE TYPE 2 ET DE PATHOLOGIES ASSOCIÉES À UN PHÉNOMÈNE D AGRÉGATION DE PROTÉINES

(30) Priority: 27.03.2008 IT MI20080514
(43) Date of publication of application: 23.02.2011
(73) Proprietor: Universita' Degli Studi di Firenze, 50121 Firenze (IT)
(72) Inventor: BERTI, Andrea, I-50121 Firenze (IT); STEFANI, Massimo, I-50010 Campi Bisenzio (IT); RIGACCI, Stefania, I-50142 Firenze (IT)
(74) Representative: Valenza, Silvia
(86) International application number: PCT/EP2009/053596
(87) International publication number: WO 2009/118380

(56) References cited:
- WO-A-2009/066021
- US-A1- 2006 177 530
- AL-AZZAWIE H F ET AL: "Hypoglycemic and antioxidant effect of oleuropein in alloxan-diabetic rabbits" 16 February 2006 (2006-02-16), LIFE SCIENCES, PERGAMON PRESS, OXFORD, GB, PAGE(S) 1371 - 1377 , XP025191171 ISSN: 0024-3205 [retrieved on 2006-02-16] left-hand column, last paragraph abstract
- DATABASE WPI Week 200650 Thomson Scientific, London, GB; AN 2006-487327 XP002537632 & KR 2005 091 672 A (JUN Y S) 15 September 2005 (2005-09-15)
- DATABASE WPI Week 200656 Thomson Scientific, London, GB; AN 2006-546573 XP002537633 & KR 2005 115 451 A (JUN Y S) 7 December 2005 (2005-12-07)

## Description

### FIELD OF THE INVENTION

This invention relates to the use of oleuropein in non-glycated form in the treatment of type 2 diabetes mellitus. Furthermore, this invention relates to the use of oleuropein in non-glycated form in the treatment of pathologies associated with protein aggregation phenomena where the pathology is type 2 diabetes mellitus. Specifically, this invention relates to the use of oleuropein in non-glycated form to prepare a pharmaceutical formula for the prophylactic and therapeutic treatment of pathologies connected to or deriving from amyloid deposit where the pathology is type 2 diabetes mellitus.

### STATE OF THE ART

It is a well known fact that type 2 non-insulin dependent diabetes mellitus (NIDDM) is marked by the presence in pancreatic islets of amyloid substance, which mainly comprises aggregates of a peptide, amylin (hIAPP).

Amyloid deposits can be seen in around 90% of NIDDM patients, and are today believed to be one of the main causes of this disease. Amylin (hIAPP) is a peptide of 37 amino acids co-secreted with the insulin by the β cells of the islets in response to a raising of blood glucose concentration. Amylin is marked by limited solubility and a high propensity to generate fibrils, through a several stage nucleation process.

Scientific research is naturally greatly involved in attempting to identify new molecules able to inhibit the formation of amyloid aggregates of hIAPP, to be used in the prophylactic and therapeutic treatment of NIDDM and in all pathologies associated with protein aggregation phenomena.

Al-Azzawie et al.; Life Science 2006, 78, 1371-1377 discloses the use of oleuropein for treating and preventing type I diabetes mellitus. Al-Azzawie et al. refers to the antioxidant effect of oleuropein in alloxan-diabetic rabbits.

US 2006/0177530 discloses the use of oleuropein for treating diabetic neuropathy.

KR 2005/0078709 and KR 2005/0107897 both disclose the use of compositions comprising oleuropein for treating a plethora of diseases (hypertension, diabetes, arteriosclerosis, viral diseases, hyperthermia, malaria, inflammation, cough, influenza, etc.) based on oleuropein antioxidant properties.

WO02009066021, discloses the use of extracts of olive oil rich in oleocanthal for the treatment of lipid metabolism conditions. In particular it is shown that oleocanthal can have a positive effect also on the activity of insulin and on glucose uptake. US2004097428 describes a method for inhibiting cancer, scar formation, disrupting the cellular cytoskeleton, and conferring resistance from infection, said method comprising the administration of oleuropein and/or its hydrolysis products.

### SUMMARY OF THE INVENTION

The subject of this invention is the use of oleuropein in non-glycated form for the preparation of a pharmaceutical formula as reported in the claims attached.

Preferred forms of this invention are explained and claimed hereto, as reported in the attached dependent claims.

### DETAILED DESCRIPTION OF THE INVENTION

The Oleaceae family includes very interesting plants, the most famous of which is the olive *(Olea Europaea subsp. Europaea).* The Oleaceae family includes 27 genera and between 400 and 900 different species.

The olive, *(Olea Europaea)* is a fruit plant, and its fruits, olives, are used for their oil. Olive oil contains many polyphenols. Oleuropein is the secoiridoid polyphenol that, in glycoside form, is characteristic of Oleaceae, and specifically of *Olea Europea*. Secoiridoids are structural derivatives of iridoids (that have a cyclopentane ring that is generally fused to a 6-membered oxygenated heterocyclic ring) for the opening of the cyclopentane ring following oxidation and breakage of a carbon-carbon bond.

Glycated oleuropein (methyl (4S,5E,6S)-4-[2-[2-(3,4-dihydroxyphenyl)ethoxy]-2-oxoethyl]-5-ethylidene-6-[(2S,3R,4S,5S,6R)-3,4,5-trihydroxy-6-(hydroxymethyl)oxan-2-yl]oxy-4H-pyran-3-carboxylate) is a single chemical species, well characterised by the molecular weight of 540.51 and the molecular formula C₂₅H₃₂O₁₃. It is hydrosoluble in glycated form, and strongly apolar in non-glycated form.

Oleuropein hydrolysis, caused by the olive glycosidases during ripening and the technological process involved in the extraction of extra-virgin olive oil, triggers the release of the aglycone that splits in the oil phase.

In our experimental system, deglycosylation has been applied by incubating 5.5 µmoles of glycated oleuropein (obtained from the company Extrasynthese) in a sodium phosphate buffer 0.1 M pH 7.0 (10 mM final) with 7.55 U.I. of β-almond glucosidase (EC 3.2.1.21). The reaction mixture was therefore centrifuged at 18,000 rpm for 10 minutes, in order to collect the oleuropein aglycone that had become insoluble in an aqueous environment. Mass spectrometer analysis of the precipitate allowed for the determination of product purity. The precipitate was fully redissolved in DMSO and stored in the dark.

The Applicant was surprised to note that the oleuropein aglycone had a great capacity to inhibit amylin amyloid (hIAPP).

The Applicant determined the aggregation kinetics of hIAPP, both in the presence and absence of oleuropein aglycone as described previously, by means of the Thioflavin T test (ThT). The ThT test is widely used to monitor the appearance of β protein and peptide rich oligomers and polymers, and is based on the increase of fluorescence at 485 nm, following excitation at 440 nm, of the ThT when it interacts with amyloid type structures. The ThT test has been carried out by using various different concentrations of oleuropein aglycone in order to generate the various molar ratios between this and the peptide.

The test was carried out by diluting the amylin (originally dissolved in 80-100%, hexafluoroisopropanol (HFIP)) in sodium phosphate buffer 10 mM pH 7.4 in the presence of 1-4% HFIP, at concentrations of between 3.125 and 12.5 µM, in the presence or absence of non-glycated oleuropein at concentrations of 0.1x, 2x, 3x and 10x of amylin concentration. The bulk solution of oleuropein aglycone, redissolved following deglycosylation, in order to obtain 100 mM in dimethyl sulfoxide (DMSO) was further diluted in the same phosphate buffer immediately prior to use, in order to obtain 100x of final concentration. Incubation was carried out at 25°C and, at various intervals, 50 µL of solution was taken from each mix, which were diluted 8 times in the glycine/NaOH buffer 0.1 M pH 8.5 containing 20 µM ThT. The fluorescence emission value was normalised each time as compared with that of a blank. Each combination of amylin and oleuropein concentrations was tested at least five times, in order to be able to calculate the standard deviation for each point.

The results show that inhibition of the amyloid aggregation of the hIAPP is dose dependent. In fact, when a non-glycated oleuropein: hIAPP ratio is used ranging between 2:1 and 10:1, according to the different preparations of hIAPP and non-glycated oleuropein, we obtain almost complete inhibition (greater than 90%) of the formation of positive ThT aggregates.

Furthermore, the Applicant also carried out a study to clarify the structural changes brought about by the presence of oleuropein during amylin aggregation.

Circular dichromism analyses were used for this, as this technique allows for the evaluation of secondary structures and the disordered structure of polymers in solution. Spectra were acquired in the region of the distant ultraviolet (between 195 and 295 nm) at 25°C, accumulating 8 successive spectra for each analysis carried out at the scanning speed of 50 nm/min. The difference against the corresponding blank spectrum was noted against each sample spectrum. In practice, immediately after having been dissolved at the concentration of 12.5µM in sodium phosphate 10 mM pH 7.4 and 1% HFIP, the amylin is found to be not very structured with a minimal presence of secondary structure. After 3 hours, the spectrum shows conversion towards a β-sheet rich structure, marked by a minimum of around 218 nm, with no change in solubility. After 24 hours, a progressive reduction in signal was noted, due to an incipient precipitation of the hIAPP present in the sample due to the reaching of critical aggregate dimensions for permanence in suspension. On the contrary, the transition to β structure, with a propensity to aggregate, is widely inhibited by the presence of the oleuropein at a concentration of 40µm. At 3 and 24 hours, a significant reduction in signal intensity can be noted, indicative of precipitation. The spectrum, however, shows no characteristics due to the presence of the β structure. Therefore, on the basis of the above-performed experimental tests, it would appear that non-glycated oleuropein interferes with the amyloid aggregation of hIAPP, inhibiting its transition to a β structure and, at the same time, causes peptide insolubility. Electronic microscope analysis of amylin aggregates obtained in the presence and absence of oleuropein has shown an amorphous precipitate forms in the presence of oleuropein. We are not yet entirely aware of the nature of the interactions between oleuropein and hIAPP. We can reasonably hypothesise that these such interactions are of a hydrophobic nature, and interfere with the interactions of the same nature that can participate in the stabilisation of intermolecular beta structures. Various experiments have also been carried out on rat insulinoma cells (the RIN-5f line) to verify protection against the toxic effect of hIAPP amyloid aggregates by oleuropein. To this end, amylin aggregates were obtained by incubating the peptide at the concentration of 3 µM in sodium phosphate 10 mM pH 7.4, 1% HFIP in the presence and absence of oleuropein aglycone 30 µM, at 25°C for 30 mins., 1 hr, 3 hrs, 5 hrs, 18 hrs, 48 hrs and 72 hrs. At the end, the aggregates were diluted 100 times in the culture medium, and administered to the cells for 24 hrs. Treatment cytotoxicity was therefore estimated as a function of the reduction of the MTT reagent (bromide of 3-[4.5-dimethylthiazol-2-yl]-2.5-diphenyltetrazolium), a test widely used to evaluate cell vitality. The colorant, administered for 2 hrs at the concentration of 0.5 mg/ml, following mitochondrial reduction, gives rise to the formation of a product that, once solubilised following cell lysis in 20% SDS, 50% *N,N* dimethylformamide, absorbs at 570 nm. Each treatment was carried out on three samples, and control treatments with only oleuropein or only culture medium were also carried out. The experiments were repeated six times, in order to carry out statistical analysis. The hIAPP aggregates show toxicity levels that decrease as time spent *in vitro* increases. Starting from 30 mins., it remains statistically significant in these conditions up to 3 hrs. The cells exposed to the hIAPP aggregates in the presence of oleuropein did not show any significant sufferance as compared with the untreated controls, and showed a significant recovery of vitality as compared with the cells treated with hIAPP alone. Treatment with oleuropein alone did not report any statistically significant differences as compared with the control. To this regard, we also carried out the treatment with larger doses of oleuropein, up to a concentration of 200 µM, and this was never found to be significantly toxic for these cells.

## Claims

1. Use of oleuropein in non-glycated form for the preparation of a pharmaceutical formulation for the treatment of pathologies associated with protein aggregation phenomena where the pathology is type 2 diabetes mellitus.

2. Use according to claim 1, where treatment is prophylactic and/or therapeutic.

3. Use according to at least one of claims 1-2, where oleuropein in non-glycated form is used in the preparation of a pharmaceutical formulation for the prophylactic and/or therapeutic treatment of type 2 diabetes mellitus.

4. Oleuropein in non-glycated form for use in the treatment of pathologies associated with protein aggregation phenomena, where the pathology is type 2 diabetes mellitus.

## Patentansprüche

1. Verwendung von Oleuropein in nicht-glykosylierter Form für die Herstellung einer pharmazeutischen Formulierung für die Behandlung von Pathologien, die mit Proteinaggregations-Phänomenen assoziiert sind, worin die Pathologie Diabetes mellitus Typ 2 ist.

2. Verwendung nach Anspruch 1, worin die Behandlung prophylaktisch und/oder therapeutisch ist.

3. Verwendung nach wenigstens einem der Ansprüche 1 bis 2, worin Oleuropein in nicht-glykosylierter Form verwendet wird bei der Herstellung einer pharmazeutischen Formulierung für die prophylaktische und/oder therapeutische Behandlung von Diabetes mellitus Typ 2.

4. Oleuropein in nicht-glykosylierter Form für die Verwendung bei der Behandlung von Pathologien, die mit Proteinaggregations-Phänomenen assoziiert sind, worin die Pathologie Diabetes mellitus Typ 2 ist.

## Revendications

1. Utilisation d'oleuropéine sous forme non glycatée pour la préparation d'une formulation pharmaceutique pour le traitement de pathologies associées aux phénomènes d'agrégation des protéines où la pathologie est le diabète sucré de type 2.

2. Utilisation selon la revendication 1, où le traitement est prophylactique et/ou thérapeutique.

3. Utilisation selon au moins l'une des revendications 1 à 2, où l'oleuropéine sous forme non glycatée est utilisée dans la préparation d'une formulation pharmaceutique pour le traitement prophylactique et/ou thérapeutique du diabète sucré de type 2.

4. Oleuropéine sous forme non glycatée à utiliser dans le traitement de pathologies associées aux phénomènes d'agrégation de protéines, où la pathologie est le diabète sucré de type 2.
